# EUROPEAN PATENT APPLICATION

(11) **EP 2 383 255 A1**
(43) Date of publication of application: **02.11.2011**
(21) Application number: 10382096.5
(22) Date of filing: 28.04.2010
(51) Int. Cl.: C07C 233/48, C07C 271/22, C07C 271/54, A61P 25/04, A61K 31/24

(54) **New compounds, synthesis and use thereof in the treatment of pain**

(71) Applicant: LACER, S.A., 08025 Barcelona (ES)
(72) Inventor: Del Castillo Nieto, Juan Carlos, 08025 Barcelona (ES); Mourelle Mancini, Marisabel, 08025 Barcelona (ES); Pubill Coy, Francisco, 08025 Barcelona (ES); Repollés Moliner, José, 08025 Barcelona (ES)
(74) Representative: ABG Patentes, S.L.

(57) **Abstract**

The present invention relates to a compound of formula (I), or pharmaceutically acceptable, stereoisomers, salts or solvates thereof 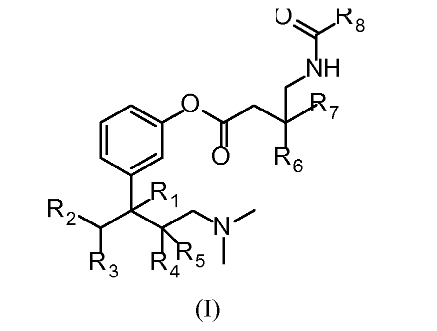 to methods for its synthesis, and use in the treatment of pain.

## Description

### FIELD OF THE INVENTION

The present invention relates to new compounds, their use for the treatment of pain, and synthesis thereof.

### BACKGROUND OF THE INVENTION

Chronic pain is a common problem that presents a major challenge to healthcare providers because of its complex natural history, unclear etiology, and poor response to therapy. The pathophysiology of chronic pain is multifactorial and complex and still is poorly understood. Some have even suggested that chronic pain might be a learned behavioral syndrome that begins with a noxious stimulus that causes pain. Patients with several psychological syndromes (e.g., major depression, somatization disorder, hypochondriasis, conversion disorder) are prone to developing chronic pain. Pain is the most common complaint that leads patients to seek medical care, and chronic pain is not uncommon. Approximately 35% of Americans have some element of chronic pain, and approximately 50 million Americans are disabled partially or totally due to chronic pain.

Various neuromuscular, reproductive, gastrointestinal, and urologic disorders may cause or contribute to chronic pain. Sometimes multiple contributing factors may be present in a single patient. The modern concept of pain treatment emphasizes the significance of prophylactic prevention of pain, as pain is more easily prevented than it is relieved. Pain is generally controlled by the administration of short acting analgesic agents, steroids and non-steroidal anti -inflammatory drugs. Analgesic agents include opiates, tricyclic antidepressants, anticonvulsivants, selective inhibitors of serotonin or noradrenalin reuptake and anti-inflammatory agents. A variety of active ingredients are available for the treatment of pain. As would be appreciated by the skilled person, therapeutics vary in pharmacological profile and effectiveness, and a large number of compounds exist which may be used for the treatment of pain, only effective in providing at most approximately a 35-50% reduction in pain.

Unfortunately, several adverse side effects are associated with the above mentioned treatments. Side effects may include, e.g., dizziness, somnolence, and harmful side effects are associated with most traditional analgesics at dosages effective for treating a neuropathy. As would be appreciated by the skill person, these side effects can include respiratory depression, disturbed sleep patterns, diminished appetite, seizures, and psychological and/or physical dependency.

For example, the pharmacological management of acute postoperative pain and chronic pain syndromes has been traditionally based on various regimens of opiates and their congeners or NSAIDs. All opiates have side effects, of which the most dangerous are respiratory and cardiovascular depression associated with excessive sedation. NSAIDs may also induce side effects such as exacerbation of bleeding tendencies and the impairment renal function.

Accordingly, there is a need to provide methods and compositions for the treatment of acute or chronic pain which provide effective control of pain with reduced harmful side effects. Clearly, there is a need for improved compositions and methods for the effective treatment of neuropathic pain.

Tramadol ((1R,2R)-rel-2-[(dimethylamino)methyl]-1-(3-methoxyphenyl) cyclohexanol) and tapentadol (3-[(1R,2R)-3-(dimethylamino)-1-ethyl-2-methylpropyl] phenol hydrochloride) are two centrally-acting analgesics for treating moderate to moderately severe pain. Despite their usefulness, both compounds have drawbacks. Among the most relevant, is the fact that they are rapidly released in the gastrointestinal tract, but their analgesic activity rapidly fades, requiring administration at short intervals. None of them shows a sufficiently good behavior in the treatment of acute pain either.

Some efforts have been made to meet these problems of tramadol and tapentadol. WO 2009/067703 proposes a controlled released formulation of tapentadol further comprising as second active agent which is tramadol, a GABA analog or an NSAID (non-steroidal anti inflammatory drugs). For example, combinations are given of tapentadol with a second active ingredient such as gabapentin or pregabalin.

Further pharmaceutical compositions comprising tramadol or tapentadol and a second analgesic have been proposed. These seek to provide analgesic effect for an extended period of time and/or increase the analgesic effect itself. Thus, WO 2009/021058 discloses a pharmaceutical composition comprising a combination of three drugs, namely, tramadol or an analog thereof, an NMDA antagonist and gabapentin or an analog thereof. US 6,562,865 reports the synergistic effect of a combined administration of tramadol and gabapentin. This effect is however unclear, and could be exclusive of certain proportions between both drugs (E.E. Codd et al. Pain, 2008, 254-262) and of the method of administration (V. Granados-Soto et al. Pharmacology, 2005, 74, 200-208).

Further combinations have been proposed without fully overcoming the above mentioned problems. This is probably due to the complex interactions which take place between different drugs, wherein different factors have to be considered (e.g. proportions between drugs and method of administration).

A different approach has been the provision of ester derivatives of the tramadol analog, O-desmethyl tramadol (3-[2-(1-Amino-1-methylethyl)-1-hydroxycyclohexyl] phenol), a known metabolite of tramadol which is even more active than tramadol itself. EP 1 251 120 Al and WO 00/27799 Al provide esters of O-desmethyl tramadol or analogs thereof, wherein the acid moiety is an aromatic group. EP 0 753 506 provides further esters of O-desmethyl tramadol. US 2005/143355 further discloses esters of O-desmethyl tramadol wherein the acid moiety is an NSAID selected from ibuprofen, naproxen, indomethacin, diclofenac, dipyron, flurbiprofen, ketoprofen and acetylsalicylic acid.

Although the above compounds and compositions represent improvements in the treatment of pain, they still do not completely overcome the existing problems of tramadol or tapentadol therapies. There is still a need to provide further compounds with analgesic effect, which can substitute or complement existing therapies. Said compounds would preferably overcome the problems described above, and they would preferably show increased or synergistic analgesic activity with reduced toxicity or side effects.

### SUMMARY OF THE INVENTION

The present invention provides more effective esters derivatives of O-desmethyl tramadol, tapentadol or analogs thereof, in the treatment of pain.

Accordingly, a first aspect of the invention is a compound of formula (I), or pharmaceutically acceptable, stereoisomers, salts or solvates thereof (compounds of the invention) 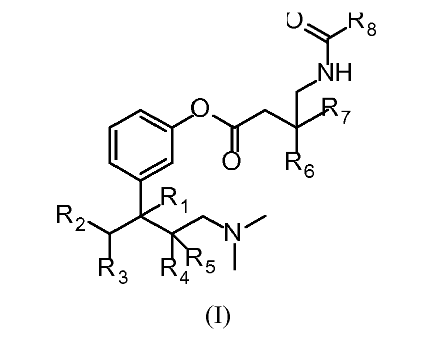 wherein
R₁ is selected from the group consisting of -H, -OH, -F, -Cl, -Br and -I;
R₂ is -H;
R₅ is -H;
or R₁ and R₂ together form a double bond and R₅ is -H
or R₁ and R₅ together form a double bond and R₂ is -H;
R₃ is a C₁-C₆ alkyl group;
R₄ is a C₁-C₆ alkyl group;
or R₃ and R₄ together form a C₂-C₄ alkylidene group;
R₆ is selected from the group consisting of -H and C₁-C₆ alkyl group;
R₇ is selected from the group consisting of -H and C₁-C₆ alkyl group;
or R₆ and R₇ together form a C₄-C₆ alkylidene group;
R₈ is selected from the group consisting of C₁-C₆ alkyl group, unsubstituted C₆-C₁₅ aryl, substituted C₆-C₁₅ aryl and O-R₉;
wherein R₉ is selected from the group consisting of C₁-C₆ alkyl group, C₆-C₁₀ aryl and C₆-C₁₀ aryl-C₁-C₂ alkyl.

A second aspect of the invention relates to a method for the synthesis of the compounds of the invention, wherein a compound of formula (II), or stereoisomers, salts or solvates thereof, reacts with a compound of formula (III), or stereoisomers, salts or solvates thereof 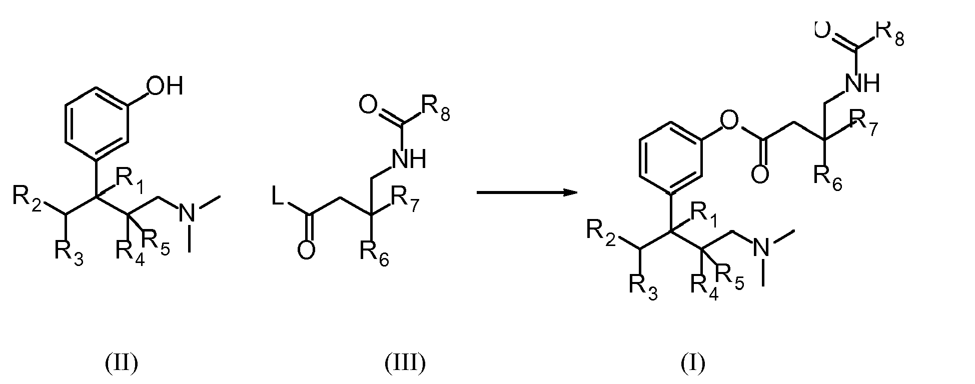 wherein R₁ to R₈ are as defined above, and L is a leaving group.

A further aspect is a compound of the invention for use as a medicament.

A further aspect is a compound of the invention for use in the treatment of pain.

A further aspect is the use of a compound of the invention for the preparation of a medicament for the treatment of pain.

A further aspect is a method of treating pain, which method comprises administering to a patient in need of such a treatment a therapeutically effective amount of at least one compound of formula (I) or a pharmaceutical composition thereof.

A further aspect is directed to a pharmaceutical composition comprising a compound of the invention and a pharmaceutically acceptable carrier.

The compounds of the invention have shown greater efficacy in the treatment of pain than other esters oftramadol previously tested. As a consequence, reduced dosages are needed which in turn would provide less side effects.

### DETAILED DESCRIPTION OF THE INVENTION

### Compounds of formula (I)

The invention also provides salts of compounds of the invention. For instance, pharmaceutically acceptable salts of compounds provided herein may be acid addition salts, base addition salts or metallic salts, and they can be synthesized from the parent compound which contains a basic or an acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, non-aqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, bisulphate, nitrate, phosphate, diacid phosphate and organic acid addition salts such as, for example, acetate, maleate, fumarate, citrate, lactate, salicilate, oxalate, succinate, tartrate, malate, mandelate, methanesulphonate (also referred to as mesilate) and p-toluenesulphonate. Examples of the alkali addition salts include inorganic salts such as, for example, ammonium, and organic alkali salts such as, for example, ethylenediamine, ethanolamine, N,N-dialkylenethanolamine, triethanolamine, glucamine and basic aminoacids salts. Examples of the metallic salts include, for example, sodium, potassium, calcium, magnesium, aluminium and lithium salts.

The term "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

It will be readily apparent to those skilled in the art that the scope of the present invention also encompasses salts which are not pharmaceutically acceptable as possible means for obtaining pharmaceutically acceptable salts.

The term "solvate" according to this invention is to be understood as meaning any form of the active compound according to the invention which has another molecule (most likely a polar solvent) attached to it via non-covalent bonding. Examples of solvates include hydrates and alcoholates, preferably C₁-C₆ alcoholates, e.g. methanolate.

It will be immediately apparent to the skilled person that the present invention encompasses all possible stereoisomers of the compounds described herein. An stereoisomer is understood by the skilled person as compounds made up of the same atoms bonded by the same sequence of bonds but having different three-dimensional structures which are not interchangeable, e.g. diastereoisomers or enantiomers. Separation methods of the different diastereoisomers or enantiomers by chemical and/or physical means are readily available to the skilled person. For example, an enantiomerically enriched mixture of enantiomers may be obtained at any stage of the synthesis by purification under chiral conditions, such as chiral supercritical fluid chromatography. Following the same methods pure enantiomers may also be isolated.

According to an embodiment of the invention, R₆ is hydrogen and R₇ is a C₁-C₆ alkyl group. According to a further embodiment of the invention, R₆ is hydrogen and R₇ is a branched C₁-C₆ alkyl group. According to a further embodiment of the invention, R₆ is hydrogen and R₇ is a C₂, branched C₃, or branched C₄ alkyl group, preferably an isopropyl group.

According to a preferred embodiment of the invention, R₆ and R₇ together form a C₄-C₆ alkylidene group, i.e. they form a C₅-C₇ cycloalkyl group together with the carbon atom to which they are attached. According to a further embodiment of the invention, R₆ and R₇ together form a C₅ alkylidene group, i.e. they form a cyclohexyl group together with the carbon atom to which they are attached.

According to an embodiment of the invention R₈ is a methyl group, a C₂-C₄ alkoxy group, a phenoxy group or a benzoxy group, preferably an ethoxy group.

In a further embodiment, R₂ is hydrogen. In a further embodiment R₃ is a C₁-C₃ alkyl group, e.g. methyl. In a further embodiment, R₁ is hydroxyl. In a further embodiment R₃ and R₄ together form a C₂, C₃ or C₄ alkylidene group. In yet a further embodiment, R₃ and R₄ form a cyclohexyl or cyclohexenyl group together with the carbon atoms to which they are attached.

A further embodiment of the invention is a compound of formula (IA), or pharmaceutically acceptable, stereoisomers, salts or solvates thereof 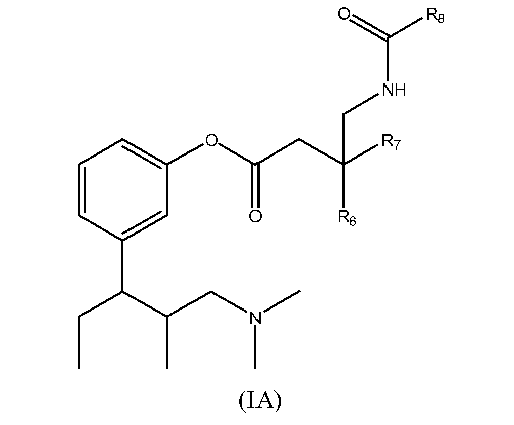 wherein R₆, R₇ and R₈ are as defined above.

A further embodiment of the invention is a compound of formula (IB), or pharmaceutically acceptable, stereoisomers, salts or solvates thereof 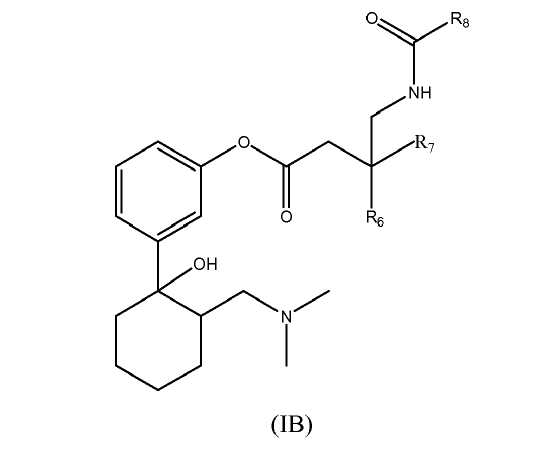 wherein R₆, R₇ and R₈ are as defined above.

A further embodiment of the invention is a compound of formula (IC), or pharmaceutically acceptable, stereoisomers, salts or solvates thereof 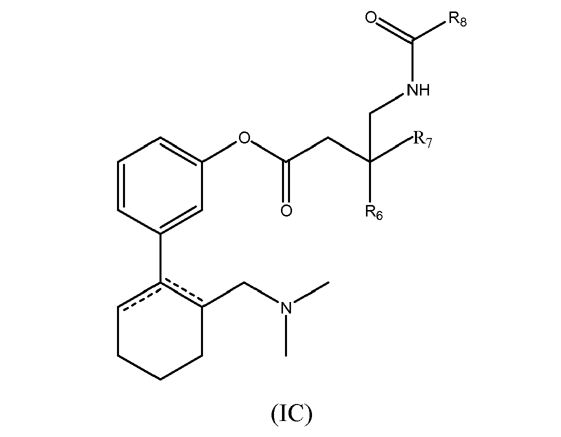 wherein R₆, R₇ and R₈ are as defined above, and one of the dotted lines may represent a double bond. Thus, compounds of formula (IC) encompass compounds of formula (ICa) and compounds of formula (ICb) as shown below:

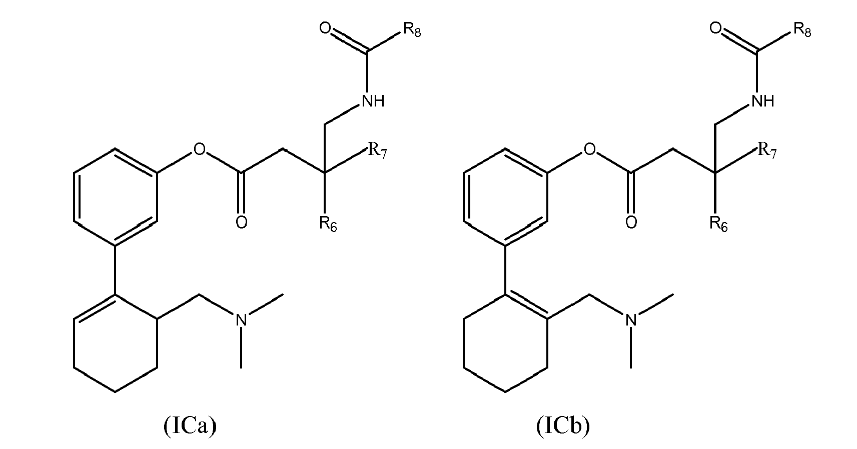

According to an embodiment, the compounds of the invention are selected from the group consisting of:
- (1R2R,1S2S)-3-(2-((dimethylamino)methyl)-1-hydroxycyclohexyl)phenyl 2-(1-((tert-butoxycarbonylamino)methyl)cyclohexyl)acetate
- 3-((1R,2R)-2-((dimethylamino)methyl)-1-hydroxycyclohexyl)phenyl 2-(1-((tert-butoxycarbonylamino)methyl)cyclohexyl)acetate
- 3-((1S,2S)-2-((dimethylamino)methyl)-1-hydroxycyclohexyl)phenyl2-(1-((tert-butoxycarbonylamino)methyl)cyclohexyl)acetate
- (1R2R,1S2S)-3-(2-((dimethylamino)methyl)-1-hydroxycyclohexyl)phenyl2-(1-((ethoxycarbonylamino)methyl)cyclohexyl)acetate
- 3-((1R,2R)-2-((dimethylamino)methyl)-1-hydroxycyclohexyl)phenyl 2-(1-((ethoxycarbonylamino)methyl)cyclohexyl)acetate
- 3-((1S,2S)-2-((dimethylamino)methyl)-1-hydroxycyclohexyl)phenyl 2-(1-((ethoxycarbonylamino)methyl)cyclohexyl)acetate
- (1R2R,1S2S)-3-(2-((dimethylamino)methyl)-1-hydroxycyclohexyl)phenyl2-(1-((methoxycarbonylamino)methyl)cyclohexyl)acetate
- (1R2R,1S2S)-3-(2-((dimethylamino)methyl)-1-hydroxycyclohexyl)phenyl2-(1-((isopropoxycarbonylamino)methyl)cyclohexyl)acetate
- (1R2R,1S2S)3-(2-((dimethylamino)methyl)-1-hydroxycyclohexyl)phenyl2-(1-((benzyloxycarbonylamino)methyl)cyclohexyl)acetate
- (1R2R,1S2S)3-(2-((dimethylamino)methyl)-1-hydroxycyclohexyl)phenyl2-(1-((phenoxycarbonylamino)methyl)cyclohexyl)acetate
- (1R2R,1S2S)3-(2-((dimethylamino)methyl)-1-hydroxycyclohexyl)phenyl 2-(1-(acetamidomethyl)cyclohexyl)acetate
- 3-(2-((dimethylamino)methyl)cyclohex-1-enyl)phenyl 2-(1-((ethoxycarbonylamino) methyl)cyclohexyl)acetate
- 3-(6-((dimethylamino)methyl)cyclohex-1-enyl)phenyl 2-(1-((ethoxycarbonylamino) methyl)cyclohexyl)acetate
- (1R2R,1S2S)3-(1-chloro-2-((dimethylamino)methyl)cyclohexyl)phenyl 2-(1-((ethoxycarbonylamino)methyl)cyclohexyl)acetate
- 3-((2R,3S)-1-(dimethylamino)-2-methylpentan-3-yl)phenyl2-(1-((ethoxycarbonyl amino)methyl)cyclohexyl)acetate
- (1R2R,1S2S)-(S)-3-(2-((dimethylamino)methyl)-1-hydroxycyclohexyl)phenyl3-((ethoxycarbonylamino)methyl)-5-methylhexanoate
- (S)-3-((1R,2R)-2-((dimethylamino)methyl)-1-hydroxycyclohexyl)phenyl 3-((ethoxycarbonylamino)methyl)-5-methylhexanoate
- (S)-3-((1S,2S)-2-((dimethylamino)methyl)-1-hydroxycyclohexyl)phenyl 3-((ethoxycarbonylamino)methyl)-5-methylhexanoate
or pharmaceutically acceptable, salts or solvates thereof.

### Synthesis of the compounds of formula (I) and intermediates of the same

As mentioned above, the compounds of the invention may be prepared by reacting a compound of formula (II), or stereoisomers, salts or solvates thereof, with a compound of formula (III), or stereoisomers, salts or solvates thereof.

Known conditions for these esterifications can be used, and appropriate conditions can be found in reference books such as chapter 10 of "Advanced Organic Chemistry: reactions, mechanisms and structures", March, J., fifth edition, Wiley-interscience (e.g. pages 484-488). Such references include preferred reaction conditions, solvents and leaving groups.

In an embodiment of the invention the solvent is an inert solvent, for example, dichloromethane, tetrahydrofurane or similar. The temperature may be comprised between -20° and 120°C, preferably between 0° y 35°C. Also, the usual reactivity enhancer for these reactions may be used, for example, carbonyldiimidazole, dicyclohexylcarbodiimide or the like. In an embodiment of the invention, catalytic amounts of a reactive enhacer, e.g. 4-dimethylaminopyridine, are used. The skilled person will change the reaction conditions or reagents depending on each specific case.

According to an embodiment of the invention L is selected from the group consisting of -OH, -F, -Cl, -Br, -I, *1H*-imidazol-1-yl, -O-R₁₀ and -OCO-R₁₁,
wherein R₁₀ is selected from the group consisting of C₁-C₆ alkyl, phenyl, and substituted phenyl; and
R₁₁, is selected from the group consisting of C₁-C₆ alkyl, phenyl, substituted phenyl, heteroaryl and substituted heteroaryl.

According to an embodiment of the invention, L is selected from the group consisting of -OH, -F, -Cl, -Br, -I.

Compounds of formula (II) can be obtained, for example, following the methods already described in NL 6610022; Flick et al. Arzneim. Forsch/Drug Res. (1978), 28 (I), 107-113; Graham R. et al. Org. Proc. Res. Dev., 2002, 6(5), 729-737; ES 2 138 271; US 3,564,100; EP 0 786 450; EP 0 693 475; WO 2008/012047.

The compounds of formula (III) can be obtained by reacting a compound of formula (IV) or stereoisomers, salts or solvates thereof, and a compound of formula (V), or stereoisomers, salts or solvates thereof. 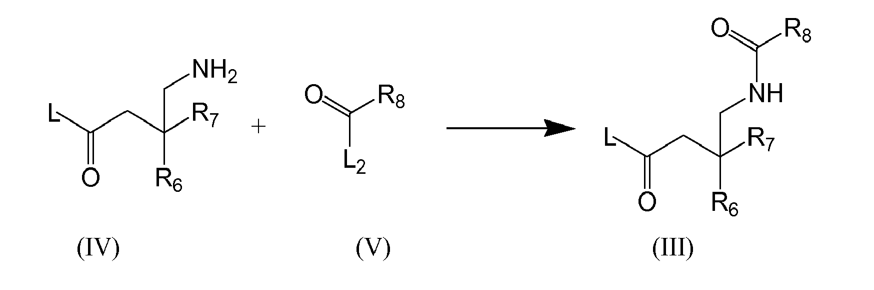 wherein R₆, R₇, and R₈ are as defined above, and L₂ has the same meaning as L above.

According to an embodiment of the invention, L₂ is -F, -Cl, -Br or -I.

The compounds of formula (IV) may be obtained following the methods described, for example, in EP 0 641 330, WO 2009/147434, US 2007/066843, US 2006/276544 or WO 2009/001372.

### Uses of the compounds of the invention

The term "treatment" in the context of this specification means administration of a compound or formulation of the invention to eliminate or ameliorate pain, or symptoms associated to pain.

Pain can be classified according to its symptoms, and different types of pain are defined in the IASP. In an embodiment of invention, pain is selected from the group consisting of allodynia, causalgia, hyperalgesia, hyperesthesia, hyperpathia, neuralgia, neuritis and neuropathy.

According to the IASP (IASP, Classification of chronic pain, 2nd Edition, IASP Press (2002), 210-212):
- "allodynia" is defined as "a pain due to a stimulus which does not normally provoke pain", .
- "causalgia" is defined as "a syndrome of sustained burning pain, allodynia and hyperpathia after a traumatic nerve lesion, often combined with vasomotor and sudomotor dysfunction and later trophic changes"
- "hyperalgesia" is defined as "an increased response to a stimulus which is normally painful
- "hyperesthesia" is defined as "increased sensitivity to stimulation, excluding the senses"
- "neuralgia" is defined as "Pain in the distribution of a nerve or nerves"
- "neuritis" is defined as "Inflammation of a nerve or nerves"
- "neuropathy" is defined as "a disturbance of function or pathological change in a nerve: in one nerve mononeuropathy, in several nerves mononeuropthy multiplex, if diffuse and bilateral, polyneuropathy".

For the purposes of the present invention, "chronic pain" is considered any pain that persists longer than the reasonable expected healing time for the involved tissues. In an embodiment of the invention it is considered ongoing pain lasting longer than 1 month, preferably longer than 2 months. In a further embodiment of the invention, "chronic pain" is considered any pain that persists longer than 3 months, preferably longer than 6 months.

According to an embodiment of the invention, the compounds of the invention are for use to eliminate the pain in patients with neuropathic pain, preferably patients who are refractory to traditional therapies.

There is no limitation to the condition causing the pain. In an embodiment the compounds of the invention are for use in the treatment of pain associated with, e.g., fibromyalgia syndrome, diabetic neuropathy, multiple sclerosis and/or cancer.

According to a further aspect, the present invention is directed to a pharmaceutical composition comprising a compound of the invention and a pharmaceutically acceptable carrier.

The term "carrier" refers to a diluent, adjuvant, excipient, or vehicle with which the active ingredient is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions, also buffers, isotonic agents or agents capable increasing solubility. Suitable pharmaceutical carriers are described in "Remington's Pharmaceutical Sciences" by E.W. Martin or "Tratado de Farmacia Galénica", C. Faulí i Trillo, Luzán 5, S.A. de Ediciones, 1993.

The pharmaceutical composition of the invention may be administered in the form of different preparations. Non limiting examples are preparations for oral administration, e.g. tablets, capsules, syrups or suspensions; ophthalmological administration, e.g. solutions, ointments or creams; and parenteral administration, e.g. aqueous and non-aqueous sterile injection solutions or aqueous and non-aqueous sterile suspensions. Also, the pharmaceutical compositions of the invention may include topical compositions, e.g. creams, ointments or pastes, or transdermic preparations such as patches or plasters. The pharmaceutical composition of the invention may also be prepared for vaginal or for rectal administration, e.g. rectal gel or suppository.

Generally an effective administered amount of a compound used in the invention will depend on the relative efficacy of the compound chosen, the severity of the disorder being treated, or the age, weight or mode of administration. However, active compounds will typically be administered once or more times a day, for example 1, 2, 3 or 4 times daily, with typical total daily doses in the range of from 0.01 to 100 mg/kg/day.

The compounds used in the present invention may also be administered with other drugs to provide a combination therapy. The other drugs may form part of the same composition, or be provided as a separate composition for administration at the same time or at different time.

### Definitions

In the definitions of the compounds described herein the following terms have the meaning indicated:
"Alkyl" refers to a straight or branched hydrocarbon chain radical consisting of carbon and hydrogen atoms, containing no unsaturation, having the number of carbon atoms indicated in each case, and which is attached to the rest of the molecule by a single bond, e. g., methyl, ethyl, n-propyl, i-propyl, n-butyl, t-butyl, n-pentyl, etc. In the absence of any indication of the number of carbon atoms it is understood that an alkyl group comprises one to twelve, preferably one to eight, more preferably one to six carbon atoms.
"Alkylidene" is understood as an alkyl group as defined above but attached to the rest of the molecule by two single bonds.
"Cycloalkyl" refers to a saturated carbocyclic ring having from the number of carbon atoms indicated in each case. In the absence of any indication of the number of carbon atoms it is understood that a cycloalkyl group comprises three to eight, preferably three to six carbon atoms. Suitable cycloalkyl groups include, but are not limited to cycloalkyl groups such as cyclopropyl, cyclobutyl, cyclopentyl or cyclohexyl.
"Aryl" refers to an aromatic hydrocarbon radical having the number of carbon atoms indicated in each case, preferably, six to ten carbon atoms, such as phenyl or naphthyl.
"Aralkyl" refers to an aryl group linked to the rest of the molecule by an alkyl group such as benzyl and phenethyl.
"Heteroaryl" refers to a stable 3- to 15- membered ring which consists of carbon atoms and from one to five heteroatoms selected from the group consisting of nitrogen, oxygen, and sulphur, preferably a 4-to 8-membered ring with one or more heteroatoms, more preferably a 5-or 6-membered ring with one or more heteroatoms, wherein at least one of the rings is aromatic. For the purposes of this invention, the heteroaryl may be a monocyclic, bicyclic or tricyclic ring system, which may include fused ring systems; and the nitrogen, carbon or sulfur atoms in the heteroaryl radical may be optionally oxidised; and the nitrogen atom may be optionally quaternized. Examples of such heteroaryls include, but are not limited to, benzimidazole, benzothiazole, furan, indole or pyridine.

Unless otherwise indicated, alkyl, cycloalkyl, alkenyl, alkynyl, aryl, aralkyl and heteroaryl radicals may be optionally substituted by one, two or three substituents such as halo, alkyl, alkenyl, alkynyl, cycloalkyl, hydroxy, alkoxy, sulfoxy, OBenzyl, OBenzoyl, carboxy, cyano, carbonyl, acyl, alkoxycarbonyl, amino, imino and nitro.

The skilled person can readily identify which substances fall under the definition of "Leaving group". For the purposes of the present invention, the term "leaving group" has its commonly accepted meaning; on page 275 of March, J. "Advanced Organic Chemistry: Reactions, Mechanism and Structure", 5th Ed., Wiley-Interscience, a leaving group is defined as the part of the molecule which becomes cleaved in the reaction. Suitable leaving groups are therefore fragments of the molecule prone to being cleaved under certain reaction conditions. They may be present in the molecule from the beginning of the reaction, or may be generated in situ. For the ester (L) and amide (L2) formation described herein, suitable leaving groups are commonly known and may be found in reference books, for example on pages 484-488, of March, J. "Advanced Organic Chemistry: Reactions, Mechanism and Structure", 5th Ed., Wiley-Interscience.

### Experimental section

The examples set forth in this description detail the suitable processes for obtaining several compounds which can be assigned to formula (I). In view of said examples, it is evident and direct for a person skilled in the art to obtain the compounds which are not explicitly exemplified, by means of applying modifications of the methods set forth, characteristic of the common general knowledge of the persons skilled in the art.

Consequently, the examples set forth below must not be interpreted as limiting the scope of the present invention, but rather as an additional and more detailed explanation which guides the person skilled in the art to a deeper understanding of the invention.

The following abbreviations are used in the following examples:
- AcOEt: Ethyl acetate
- AcOH: Acetic acid
- DMSO-d₆: Hexadeuterodimethyl sulfoxide
- EtOH: Ethanol
- Et₂O: Diethyl ether
- HPLC: high pressure liquid chromatography
- IPA: Isopropyl alcohol
- RT: Room temperature
- THF: Tetrahydrofuran
- TLC: Thin layer chromatography
- e.e.: Enantiomeric Excess

The nuclear magnetic resonance spectra have been carried out in a Varian Gemini-200 apparatus.

The ¹H -NMR spectra indicate the working frequency and the solvent used to make the spectrum. The position of the signals is indicated in δ (ppm), using the signal of the protons of the solvent as reference. The reference values are 7.24 ppm for chloroform and 2.49 ppm for deuterated dimethyl sulfoxide. Within brackets are indicated the number of protons corresponding to each signal measured by electronic integration and the type of signal using the following abbreviations: s (singlet), d (doublet), t (triplet), q (quadruplet), s.b. (signal broad). The signal assignation is indicated in some cases.

The ¹³C-NMR spectra indicate the working frequency and the solvent used to make the spectrum. The position of the signals is indicated in δ (ppm), using the central signal of the solvent as reference. The reference values are 77.00 ppm for chloroform and 39.50 ppm for deuterated dimethylsulfoxide.

The determination of impurities is performed by HPLC using an HPLC apparatus equipped with a photodiode array detector (scan from 200 to 450 nm, and then extract chromatogram at 201 nm). Chromatographic separation is performed using an Atlantis RPC18 column, 150 x 4.6 mm, 5 µm, at 30 °C, with the following gradient method:

| t/min | A/% | B/% |
|---|---|---|
| 0 | 100 | 0 |
| 20 | 5 | 95 |
| 21 | 100 | 0 |
| 30 | 100 | 0 |

where A is 5 mM ammonium formate at pH 3 and B is acetonitrile. The flow rate is 1 ml·min⁻¹, and the compartment of the samples is set at 4 °C.

Samples are prepared at 1 mg·ml⁻¹: 1.5 mg of sample are weighed, then are dissolved with 1ml of acetonitrile, and finally are added 0.5 ml of ammonium formate 5 mM at pH 3.

### A) SYNTHESIS

### Example 1. (1R2R, 1S2S)-3-(2-((dimethylamino)methyl)-1-hydroxycyclohexyl) phenyl 2-(1-((tert-butoxycarbonylamino)methyl)cyclohexyl)acetate

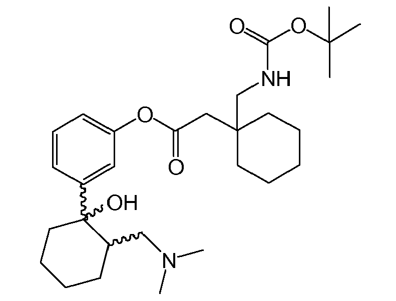

To a solution of 2,7g (10 mmol) of 2-(1-((tert-butoxycarbonylamino) methyl)cyclohexyl) acetic acid (2-[1-(tert-Butoxycarbonylamino-methyl)-cyclohexyl]-acetic acid), obtained as described in US2006046967, in 200 ML of dichloromethane and 3 ML of N,N-Dimethylformamide were added 2,5g (10 mmol) of (1R2R, 1S2S)3-(2-((dimethylamino)methyl)-1-hydroxycyclohexyl)phenol, 6,2 g (30 mmol) of N,N-dicyclohexylcarbodiimide (Fluka 36650) and 36 mg (0.3 mmol) of 4-dimethylaminopyridine (Fluka 39405).

The reaction was stirred at RT for 16 h. The white solid was filtered, and the filtrated liquids evaporated under reduced pressure. The resulting residue was re-dissolved in AcOEt (100 ML) and washed with 3x50 ML of brine. The organic phase was dried with sodium sulfate, filtered and evaporated under reduced pressure. The residue was purified over silica gel, by first eluting with dichloromethane, then with dichloromethane/EtOH 98/2, and finally with dichloromethane/EtOH 95/5 (at 90/10 the compound was obtained with small amounts of dicyclohexylurea).

The fractions containing the title compound where combined and concentrated to dryness, and then treated with 30 ML of dichloromethane. The resulting precipitated dicyclohexylurea was filtered. Finally, the liquids were evaporated to dryness under reduced pressure to yield 2,2 g (44%) of the title compound as a colorless oil.

### Purity HPLC: 98%

**¹H -NMR (DMSO-d₆, 200 MHz):** 7,31 (b.s., 1H,); 7,29 (s, 1H,); 7,19 (b.s., 1H,); 6,90-6,80 (m., 1H,); 6,80-6,70 (t., 1H, NH); 5,20-4,80 (b.s., 1H, OH); 3,09 (d, 2H, J=6,2 Hz, NCH₂); 2,47 (s, 2H, CH₂CO₂); 2,20-2,00 (m, 1H,); 1,88 (s, 6H, NCH₃); 2,00-1,20 (m, 20H); 1,37 (s, 9H, BOC)

**¹³C-NMR (DMSO-d₆, 50 MHz):** 170,05 (1C, C=O); 156,43 (1C, C=O); 151,67 (1C, C=C-O); 150,19 (1C, COH-C=CH); 128,43 (1C, phenyl); 122,16 (1C, phenyl); 119,14 (1C, phenyl); 118,52 (1C, phenyl); 77,50 (1C, C-O); 74,45 (1C, C-OH); 60,35 (1C, CH₂N); 46,13 (1C, CH₂NH); 45,86 (2C, NCH₃); 43,15 (1C); 40,97 (1C); 39,50 (1C); 37,14 (1C); 32,54 (2C); 28,22 (3C, CH₃); 26,38 (1C); 25,56 (2C); 21,69 (1C); 21,04 (2C);

### Example 2. 3-((1R,2R)-2-((dimethylamino)methyl)-1-hydroxycyclohexyl)phenyl 2-(1-((tert-butoxycarbonylamino)methyl)cyclohexyl)acetate

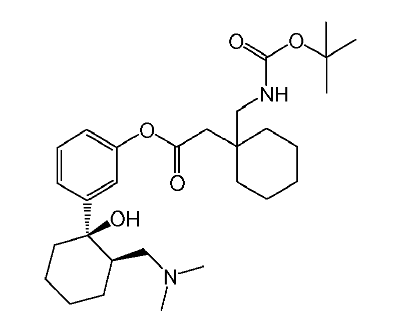

Following the procedure described in Example 1, but using as starting material the 1R2R isomer of 3-(2-((dimethylamino)methyl)-1-hydroxycyclohexyl)phenol, 2,5 g of the title compound were obtained as a colorless oil (50%) with a 95% purity (HPLC) and 100% e.e.

**¹H -NMR (DMSO-d₆, 200 MHz):** 7,31 (b.s., 1H,); 7,29 (s, 1H,); 7,19 (b.s., 1H,); 6,90-6,80 (m., 1H,); 6,80-6,70 (t., 1H, NH); 5,20-4,80 (b.s., 1H, OH); 3,09 (d, 2H, J=6,2 Hz, NCH₂); 2,47 (s, 2H, CH₂CO₂); 2,20-2,00 (m, 1H,); 1,88 (s, 6H, NCH₃); 2,00-1,20 (m, 20H); 1,37 (s, 9H, BOC)

**¹³C-NMR (DMSO-d₆, 50 MHz):** 170,05 (1C, C=O); 156,43 (1C, C=O); 151,67 (1C, C=C-O); 150,19 (1C, COH-C=CH); 128,43 (1C, phenyl); 122,16 (1C, phenyl); 119,14 (1C, phenyl); 118,52 (1C, phenyl); 77,50 (1C, C-O); 74,45 (1C, C-OH); 60,35 (1C, CH₂N); 46,13 (1C, CH₂NH); 45,86 (2C, NCH₃); 43,15 (1C); 40,97 (1C); 39,50 (1C); 37,14 (1C); 32,54 (2C); 28,22 (3C, CH₃); 26,38 (1C); 25,56 (2C); 21,69 (1C); 21,04 (2C);

### Example 3. 3-((1S,2S)-2-((dimethylamino)methyl)-1-hydroxycyclohexyl)phenyl 2-(1-((tert-butoxycarbonylamino)methyl)cyclohexyl)acetate

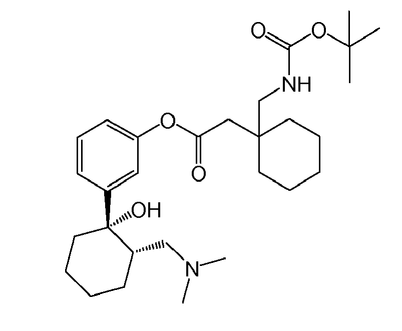

Following the procedure described in Example 1, but using as starting material the 1S2S isomer of 3-(2-((dimethylamino)methyl)-1-hydroxycyclohexyl)phenol, 2,45 g of the title compound were obtained as a colorless oil (49%) with a 99,2% purity (HPLC) and 98,8% e.e. (a^{25°C}_{D}= -17,8° (c=1, MeOH)).

**¹H -NMR (DMSO-d₆, 200 MHz):** 7,31 (b.s., 1H,); 7,29 (s, 1H,); 7,19 (b.s., 1H,); 6,90-6,80 (m., 1H,); 6,80-6,70 (t., 1H, NH); 5,20-4,80 (b.s., 1H, OH); 3,09 (d, 2H, J=6,2 Hz, NCH₂); 2,47 (s, 2H, CH₂CO₂); 2,20-2,00 (m, 1H,); 1,88 (s, 6H, NCH₃); 2,00-1,20 (m, 20H); 1,37 (s, 9H, BOC)

**¹³C-NMR (DMSO-d₆, 50 MHz):** 170,05 (1C, C=O); 156,43 (1C, C=O); 151,67 (1C, C=C-O); 150,19 (1C, COH-C=CH); 128,43 (1C, phenyl); 122,16 (1C, phenyl); 119,14 (1C, phenyl); 118,52 (1C, phenyl); 77,50 (1C, C-O); 74,45 (1C, C-OH); 60,35 (1C, CH₂N); 46,13 (1C, CH₂NH); 45,86 (2C, NCH₃); 43,15 (1C); 40,97 (1C); 39,50 (1C); 37,14 (1C); 32,54 (2C); 28,22 (3C, CH₃); 26,38 (1C); 25,56 (2C); 21,69 (1C); 21,04 (2C);

### Example 4. (1R2R, 1S2S) 3-(2-((dimethylamino)methyl)-1-hydroxycyclohexyl) phenyl 2-(1-((ethoxycarbonylamino)methyl)cyclohexyl)acetate

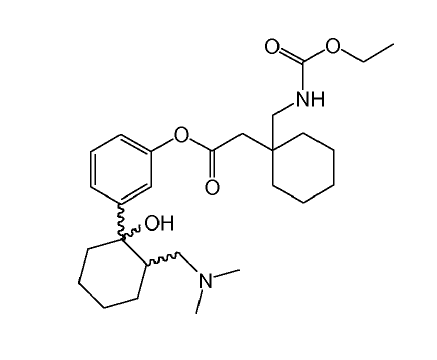

### 4a) Synthesis of 2-(1-((ethoxycarbonylamino)methyl)cyclohexyl)acetic acid

8,56 g (50 mmol) of 2-[1-(aminomethyl)cyclohexyl]acetic acid in 200 ML of dichloromethane were placed in a three neck flask having a thermometer and an addition funnel. After cooling the solution to 0°C, 14 ML (100 mmol) of triethylamine and 12,6 ML (100 mmol) trimethylchlorosilane were added. The mixture was allowed to warm to RT and stirring continued for 15 more minutes. Then it was cooled to 0°C and 5,26 ml (55 mmol) of ethylchloroformiate in 50 ML of dichloromethane were added.

The reaction was allowed to reach RT and stirring continued for 16 h, after which it was washed with 2x50 ML of brine and with 50 ML of HCl 1N. The organic phase was washed over Na₂SO₄, filtered and the liquids concentrated to dryness. The residue was purified over silica gel, eluting with a 1/1 mixture of Hexane/AcOEt. 8,3 g (69%) were obtained as a colorless oil which crystallized over time.

**¹H -NMR (DMSO-d₆, 200 MHz):** 12,10-11,80 (b.s., 1H, CO₂H); 6,84 (t., 1H, NH); 3,95 (q., 2H, J=7 Hz, CH₂O); 3,06 (d, 2H, J=6,4 Hz, NCH₂); 2,15 (s, 2H, CH₂CO₂); 1,50-1,20 (m, 10H); 1,12 (t, 3H, CH₃)

**¹³C-NMR (DMSO-d₆, 50 MHz):** 173,17 (1C, C=O); 156,85 (1C, C=O); 59,62 (1C, CO); 46,87 (1C, CH₂NH); 40,16 (1C, CH₂CO₂); 36,64 (1C); 32,39 (2C); 25,58 (1C); 21,09 (2C); 14,67 (1C, CH₃)

### 4b) Synthesis of (1R2R, 1S2S) 3-(2-((dimethylamino)methyl)-1-hydroxy cyclohexyl)phenyl 2-(1-((ethoxycarbonylamino)methyl)cyclohexyl)acetate

Following the procedure described in Example 1, but using as starting material the product of Example 4a), 1.9 g of the title compound were obtained as a colorless oil (40%) with a 95% purity (HPLC).

**¹H -NMR (DMSO-d₆, 200 MHz):** 7,25 (b.s., 2H,); 7,13 (b.s., 1H,); 7,10-6,90 (m., 1H,); 6,86 (b.s., 1H, NH); 3,94 (q., 2H, J=7 Hz, OCH₂); 3,11 (d, 2H, J=6,2 Hz, NCH₂); 2,46 (s, 2H, CH₂CO₂); 2,10-1,90 (m, 1H,); 1,85 (s, 6H, NCH₃); 2,00-1,20 (m, 20H); 1,10 (t, 3H, CH₃)

**¹³C-NMR (DMSO-d₆, 50 MHz):** 170,08 (1C, C=O); 156,91 (1C, C=O); 151,72 (1C, C=C-O); 150,18 (1C, COH-C=CH); 128,54 (1C, phenyl); 122,22 (1C, phenyl); 119,14 (1C, phenyl); 118,51 (1C, phenyl); 74,49 (1C, C-OH); 60,38 (1C, CH₂N); 59,64 (1C, CH₂O); 46,13 (1C, CH₂NH); 45,86 (2C, NCH₃); 43,14 (1C); 40,99 (1C); 39,50 (1C); 37,19 (1C); 32,51 (2C); 26,41 (1C); 25,56 (2C); 21,71 (1C); 21,06 (2C); 14,66 (1C, CH₃)

### Example 5. 3-((1R,2R)-2-((dimethylamino)methyl)-1-hydroxycyclohexyl)phenyl 2-(1-((ethoxycarbonylamino)methyl)cyclohexyl)acetate

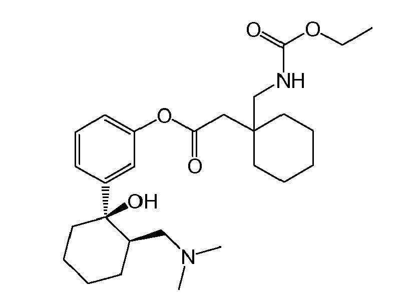

Following the procedure described in Example 1, but using as starting material the product of Example 4a), and reacting it with the 1R2R isomer of 3-(2-((dimethylamino)methyl)-1-hydroxycyclohexyl)phenol, 2.5 g of the title compound were obtained as a colorless oil (53%) with a 98% purity (HPLC) and 100% e.e.

**¹H -NMR (DMSO-d₆, 200 MHz):** 7,25 (b.s., 2H,); 7,13 (b.s., 1H,); 7,10-6,90 (m., 1H,); 6,86 (b.s., 1H, NH); 5,10-4,90 (b.s., 1H, OH); 3,99 (q., 2H, J=7 Hz, OCH₂); 3,11 (d, 2H, J=6,2 Hz, NCH₂); 2,46 (s, 2H, CH₂CO₂); 2,10-1,90 (m, 1H,); 1,85 (s, 6H, NCH₃); 2,00-1,20 (m, 20H); 1,10 (t, 3H, CH₃)

**¹³C-NMR (DMSO-d₆, 50 MHz):** 170,08 (1C, C=O); 156,91 (1C, C=O); 151,72 (1C, C=C-O); 150,18 (1C, COH-C=CH); 128,54 (1C, phenyl); 122,22 (1C, phenyl); 119,14 (1C, phenyl); 118,51 (1C, phenyl); 74,49 (1C, C-OH); 60,38 (1C, CH₂N); 59,64 (1C, CH₂O); 46,13 (1C, CH₂NH); 45,86 (2C, NCH₃); 43,14 (1C); 40,99 (1C); 39,50 (1C); 37,19 (1C); 32,51 (2C); 26,41 (1C); 25,56 (2C); 21,71 (1C); 21,06 (2C); 14,66 (1C, CH₃)

### Example 6. 3-((1S,2S)-2-((dimethylamino)methyl)-1-hydroxycyclohexyl)phenyl 2-(1-((ethoxycarbonylamino)methyl)cyclohexyl)acetate

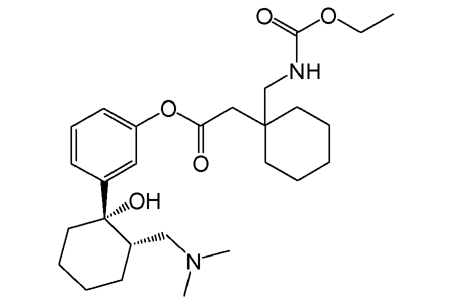

Following the procedure described in Example 1, but using as starting material the product of Example 4a), and reacting it with the 1S2S isomer of 3-(2-((dimethylamino)methyl)-1-hydroxycyclohexyl)phenol, 1.8 g of the title compound were obtained as a colorless oil (38%) with a 97.6% purity (HPLC) and 100% e.e.

**¹H -NMR (DMSO-d₆, 200 MHz):** 7,25 (b.s., 2H,); 7,13 (b.s., 1H,); 7,10-6,90 (m., 1H,); 6,86 (b.s., 1H, NH); 5,10-4,90 (b.s., 1H, OH); 3,99 (q., 2H, J=7 Hz, OCH₂); 3,11 (d, 2H, J=6,2 Hz, NCH₂); 2,46 (s, 2H, CH₂CO₂); 2,10-1,90 (m, 1H,); 1,85 (s, 6H, NCH₃); 2,00-1,20 (m, 20H); 1,10 (t, 3H, CH₃)

**¹³C-NMR (DMSO-d₆, 50 MHz):** 170,08 (1C, C=O); 156,91 (1C, C=O); 151,72 (1C, C=C-O); 150,18 (1C, COH-C=CH); 128,54 (1C, phenyl); 122,22 (1C, phenyl); 119,14 (1C, phenyl); 118,51 (1C, phenyl); 74,49 (1C, C-OH); 60,38 (1C, CH₂N); 59,64 (1C, CH₂O); 46,13 (1C, CH₂NH); 45,86 (2C, NCH₃); 43,14 (1C); 40,99 (1C); 39,50 (1C); 37,19 (1C); 32,51 (2C); 26,41 (1C); 25,56 (2C); 21,71 (1C); 21,06 (2C); 14,66 (1C, CH₃)

### Example 7. (1R2R, 1S2S) 3-(2-((dimethylamino)methyl)-1-hydroxycyclohexyl) phenyl 2-(1-((methoxycarbonylamino)methyl)cyclohexyl)acetate

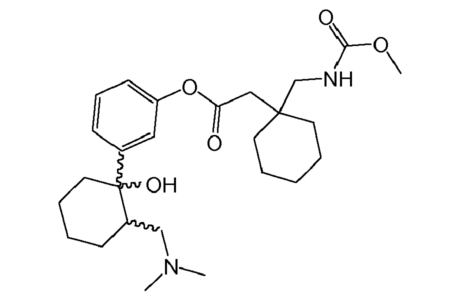

### 7a) Synthesis of 2-(1-((methoxycarbonylamino)methyl)cyclohexyl)acetic acid

Following the procedure described in Example 4a, but using methylchloroformiate instead of ethylchloroformiate, the title compound was obtained as a colorless oil which crystallized over time.

**¹H -NMR (DMSO-d₆, 200 MHz):** 12,10-11,80 (b.s., 1H, CO₂H); 6,84 (t., 1H, NH); 3,50 (s., 3H, CH₃O); 3,07 (d, 2H, J=6,2 Hz, NCH₂); 2,15 (s, 2H, CH₂CO₂); 1,50-1,20 (m, 10H);

**¹³C-NMR (DMSO-d₆, 50 MHz):** 173,16 (1C, C=O); 157,28 (1C, C=O); 51,27 (1C, OCH₃); 46,95 (1C, CH₂NH); 40,16 (1C, CH₂CO₂); 36,65 (1C); 32,34 (2C); 25,57 (1C); 21,07 (2C);

### 7b) Synthesis of (1R2R, 1S2S) 3-(2-((dimethylamino)methyl)-1-hydroxy cyclohexyl)phenyl 2-(1-((methoxycarbonylamino)methyl)cyclohexyl)acetate

Following the procedure described in Example 1, but using as starting material the product of Example 7a), 1.5 g of the title compound were obtained as a colorless oil (43%) with a 93.4% purity (HPLC).

**¹H -NMR (DMSO-d₆, 200 MHz):** 7,30 (b.s., 2H,); 7,17 (b.s., 1H,); 7,10-7,00 (m., 1H,); 6,86 (b.s., 1H, NH); 5,10-4,80 (b.s., 1H, OH); 3,53 (s., 3H, OCH₃); 3,16 (d, 2H, J=6,2 Hz, NCH₂); 2,49 (s, 2H, CH₂CO₂); 2,20-2,00 (m, 1H,); 1,89 (s, 6H, NCH₃); 2,00-1,00 (m, 20H).

**¹³C-NMR (DMSO-d₆, 50 MHz):** 170,08 (1C, C=O); 156,91 (1C, C=O); 151,72 (1C, C=C-O); 150,15 (1C, COH-C=CH); 128,54 (1C, phenyl); 122,22 (1C, phenyl); 119,12 (1C, phenyl); 118,49 (1C, phenyl); 74,47 (1C, C-OH); 60,38 (1C, CH₂N); 51,28 (1C, OCH₃); 46,13 (1C, CH₂NH); 45,86 (2C, NCH₃); 43,14 (1C); 40,99 (1C); 39,50 (1C); 37,20 (1C); 32,45 (2C); 26,41 (1C); 25,56 (2C); 21,71 (1C); 21,04 (2C);

### Example 8. (1R2R, 1S2S) 3-(2-((dimethylamino)methyl)-1-hydroxycyclohexyl) phenyl 2-(1-((isopropoxycarbonylamino)methyl)cyclohexyl)acetate

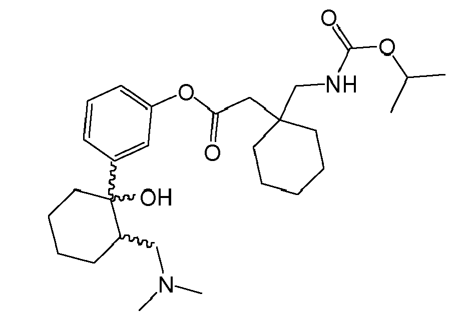

### 8a) Synthesis of 2-(1-((isopropoxycarbonylamino)methyl)cyclohexyl)acetic acid

Following the procedure described in Example 4a, but using isopropylchloroformiate instead of ethylchloroformiate, the title compound was obtained as a colorless oil.

**¹H -NMR (DMSO-d₆, 200 MHz):** 12,10-11,80 (b.s., 1H, CO₂H); 6,84 (t., 1H, NH); 4,80-4,60 (m, 1H, CH); 3,05 (d, 2H, J=6,2 Hz, NCH₂); 2,15 (s, 2H, CH₂CO₂); 1,50-1,20 (m, 10H); 1,14 (d, 6H, 2 CH₃)

**¹³C-NMR (DMSO-d₆, 50 MHz):** 173,17 (1C, C=O); 156,46 (1C, C=O); 66,62 (1C, OCH); 46,77 (1C, CH₂NH); 40,16 (1C, CH₂CO₂); 36,65 (1C); 32,38 (2C); 25,59 (1C); 22,07 (2C); 21,09 (2C)

### 8b) Synthesis of (1R2R, 1S2S)3-(2-((dimethylamino)methyl)-1-hydroxycyclo hexyl)phenyl 2-(1-((isopropoxycarbonylamino)methyl)cyclohexyl) acetate

Following the procedure described in Example 1, but using as starting material the product of Example 8a), the title compound was obtained as a colorless oil (40%).

**¹H -NMR (DMSO-d₆, 200 MHz):** 7,30 y 7,29 (d., 2H,); 7,17 (b.s., 1H,); 7,05-6,95 (t., 1H, NH); 6,90-6,80 (b.s., 1H); 5,10-4,80 (b.s., 1H, OH); 4,80-4,60 (m., 1H, OCH); 3,14 (d, 2H, J=6,2 Hz, NCH₂); 2,49 (s, 2H, CH₂CO₂); 2,20-2,00 (m, 1H,); 1,89 (s, 6H, NCH₃); 2,00-1,20 (m, 20H); 1,15 (d, 6H, J=6,2 Hz, 2 CH₃)

**¹³C-NMR (DMSO-d₆, 50 MHz):** 170,08 (1C, C=O); 156,51 (1C, C=O); 151,69 (1C, C=C-O); 150,18 (1C, COH-C=CH); 128,51 (1C, phenyl); 122,20 (1C, phenyl); 119,17 (1C, phenyl); 118,51 (1C, phenyl); 74,45 (1C, C-OH); 66,65 (1C, OCH); 60,38 (1C, CH₂N); 46,54 (1C, CH₂NH); 45,83 (2C, NCH₃); 43,14 (1C); 40,99 (1C); 39,50 (1C); 37,16 (1C); 32,51 (2C); 26,39 (1C); 25,54 (2C); 22,06 (2C, CH₃); 21,70 (1C); 21,06 (2C);

### Example 9. (1R2R, 1S2S)3-(2-((dimethylamino)methyl)-1-hydroxycyclohexyl) phenyl 2-(1-((benzyloxycarbonylamino)methyl)cyclohexyl)acetate

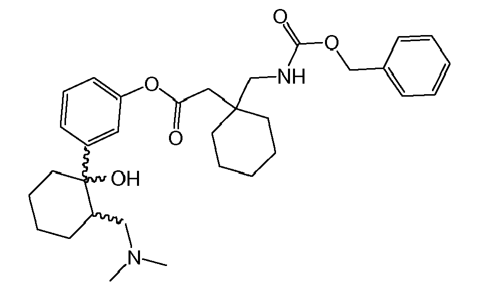

### 9a) Synthesis of 2-(1-((benzyloxycarbonylamino)methyl)cyclohexyl)acetic acid

Following the procedure described in Example 4a, but using benzylchloroformiate instead of ethylchloroformiate, the title compound was obtained as a very dense colorless oil. Yield 56%

**¹H -NMR (DMSO-d₆, 200 MHz):** 12,00 (s., 1H, CO₂H); 7,35-7,31 (m, 5H, Ph); 7,05 (t., 1H, NH); 5,01 (s, 2H, OCH₂); 3,11 (d, 2H, J=6,2 Hz, NCH₂); 2,18 (s, 2H, CH₂CO₂); 1,50-1,20 (m, 10H)

**¹³C-NMR (DMSO-d₆, 50 MHz):** 173,07 (1C, C=O); 156,69 (1C, C=O); 137,31 (1C,phenyl); 128,31 (2C,phenyl); 127,68 (1C,phenyl); 127,62 (2C,phenyl); 65,23 (1C, OCH₂); 46,95 (1C, CH₂NH); 40,83 (1C, CH₂CO₂); 36,62 (1C); 32,37 (2C); 25,55 (1C); 21,06 (2C)

### 9b) Synthesis of (1R2R, 1S2S)3-(2-((dimethylamino)methyl)-1-hydroxycyclo hexyl)phenyl 2-(1-((benzyloxycarbonylamino)methyl)cyclohexyl) acetate

Following the procedure described in Example 1, but using as starting material the product of Example 9a), he title compound was obtained as a colorless oil (43%) with a 95.5% purity (HPLC).

**¹H -NMR (DMSO-d₆, 200 MHz):** 7,33-7,20 (m., 8H,); 7,18 (b.s., 1H,); 6,85-6,80 (b.s., 1H, NH); 5,04 (b.s., 3H, OCH₂+OH); 3,18 (d, 2H, J=6,2 Hz, NCH₂); 2,49 (s, 2H, CH₂CO₂); 2,20-2,00 (m, 1H,); 1,89 (s, 6H, NCH₃); 2,00-1,20 (m, 20H)

**¹³C-NMR (DMSO-d₆, 50 MHz):** 170,08 (1C, C=O); 156,51 (1C, C=O); 151,69 (1C, C=C-O); 150,18 (1C, COH-C=CH); 137,22 (1C, phenyl); 128,49 (1C, phenyl); 128,29 (2C, phenyl); 127,70 (3C, phenyl); 122,17 (1C, phenyl); 119,11 (1C, phenyl); 118,47 (1C, phenyl); 74,45 (1C, C-OH); 65,24 (1C, OCH₂); 60,35 (1C, CH₂N); 46,68 (1C, CH₂NH); 45,84 (2C, NCH₃); 43,14 (1C); 40,99 (1C); 39,50 (1C); 37,16 (1C); 32,45 (2C); 26,37 (1C); 25,51 (2C); 21,67 (1C); 21,02 (2C);

### Example 10. (1R2R, 1S2S)3-(2-((dimethylamino)methyl)-1-hydroxycyclohexyl) phenyl 2-(1-((phenoxycarbonylamino)methyl)cyclohexyl)acetate

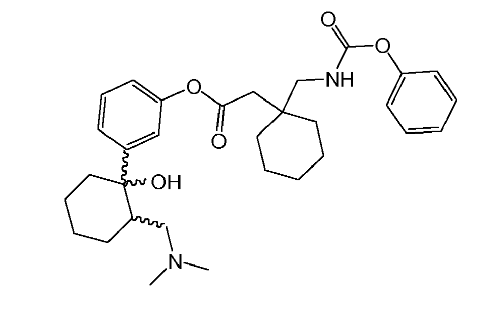

### 10a) Synthesis of 2-(1-((fenoxycarbonylamino)methyl)cyclohexyl)acetic acid

Following the procedure described in Example 4a, but using phenylchloroformiate instead of ethylchloroformiate, the title compound was obtained as a very dense colorless oil. Yield 75% **¹H -NMR (DMSO-d₆, 200 MHz):** 12,00 (s., 1H, CO₂H); 7,60 (t., 1H, NH); 7,40-7,31 (m, 2H, Ph); 7,21-7,06 (m, 3H, Ph); 3,18 (d, 2H, J=6,2 Hz, NCH₂); 2,24 (s, 2H, CH₂CO₂); 1,50-1,20 (m, 10H)

**¹³C-NMR (DMSO-d₆, 50 MHz):** 173,11 (1C, C=O); 154,98 (1C, C=O); 151,21 (1C, CO); 129,22 (2C, phenyl); 124,81 (1C, phenyl); 121,73 (2C, phenyl); 47,20 (1C, CH₂NH); 40,83 (1C, CH₂CO₂); 36,66 (1C); 32,41 (2C); 25,57 (1C); 21,09 (2C)

### 10b) Synthesis of (1R2R, 1S2S)3-(2-((dimethylamino)methyl)-1-hydroxycyclo hexyl)phenyl 2-(1-((fenoxycarbonylamino)methyl)cyclohexyl) acetate

Following the procedure described in Example 1, but using as starting material the product of Example 10a), the title compound was obtained as a colorless oil (49%) with a 94.2% purity (HPLC).

**¹H -NMR (DMSO-d₆, 200 MHz):** 7,80 (t., 1H, NH); 7,39-7,31 (m., 4H,); 7,20-7,07 (m., 4H,); 6,90 (b.s., 1H,); 5,20-4,80 (b.s., 1H, OH); 3,27 (d, 2H, J=6,2 Hz, NCH₂); 2,60 (s, 2H, CH₂CO₂); 2,20-2,00 (m, 1H,); 1,89 (s, 6H, NCH₃); 2,00-1,20 (m, 20H)

**¹³C-NMR (DMSO-d₆, 50 MHz):** 170,01 (1C, C=O); 156,04 (1C, C=O); 151,69 (1C, C=C-O); 151,19 (1C, C-O); 150,18 (1C, COH-C=CH); 129,16 (2C, phenyl); 128,49 (1C, phenyl); 124,77 (1C, phenyl); 122,21 (1C, phenyl); 121,64 (2C, phenyl); 119,12 (1C, phenyl); 118,51 (1C, phenyl); 74,52 (1C, C-OH); 60,40 (1C, CH₂N); 47,01 (1C, CH₂NH); 45,89 (2C, NCH₃); 43,17 (1C); 40,99 (1C); 39,50 (1C); 37,20 (1C); 32,57 (2C); 26,43 (1C); 25,55 (2C); 21,71 (1C); 21,06 (2C);

### Example 11. (1R2R, 1S2S)3-(2-((dimethylamino)methyl)-1-hydroxycyclohexyl) phenyl 2-(1-(acetamidomethyl)cyclohexyl)acetate

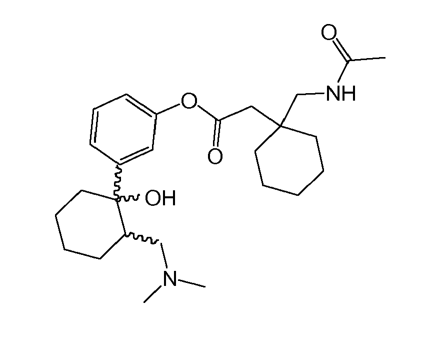

Following the procedure described in Example 1, but using as starting material 2-(1-(acetamidomethyl)cyclohexyl)acetic acid obtained as described in WO2001042191, the title compound was obtained as a colorless oil with a 96.3% purity (HPLC).

**¹H -NMR (DMSO-d₆, 200 MHz):** 7,70 (b.s., 1H, NH); 7,28 (b.s., 2H,); 7,17 (b.s., 1H,); 6,89 (b.s., 1H,); 5,10-4,80 (b.s., 1H, OH); 3,20 (d, 2H, J=6,2 Hz, NCH₂); 2,50 (s, 2H, CH₂CO₂); 2,20-2,00 (m, 1H,); 1,88 (s, 6H, NCH₃); 1,84 (s, 3H, COCH₃); 2,00-1,00 (m, 20H).

**¹³C-NMR (DMSO-d₆, 50 MHz):** 170,08 (1C, C=O); 169,67 (1C, C=O); 151,76 (1C, C=C-O); 150,18 (1C, COH-C=CH); 128,55 (1C, phenyl); 122,22 (1C, phenyl); 119,14 (1C, phenyl); 118,49 (1C, phenyl); 74,50 (1C, C-OH); 60,40 (1C, CH₂N); 45,90 (2C, NCH₃); 44,42 (1C, CH₂NH); 43,19 (1C); 41,02 (1C); 39,50 (1C); 37,11 (1C); 32,67 (2C); 26,44 (1C); 25,58 (2C); 22,63 (1C, CH₃); 21,74 (1C); 21,10 (2C);

### Example 12. 3-(2-((dimethylamino)methyl)cyclohex-1-enyl)phenyl 2-(1-((ethoxy carbonylamino)methyl)cyclohexyl)acetate

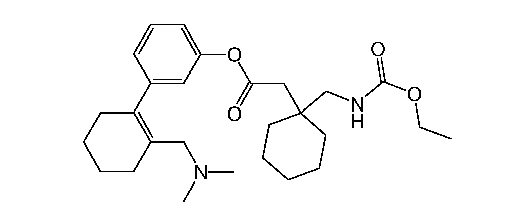

Following the procedure described in Example 1, but using as starting material 3-(2-((dimethylamino)methyl)cyclohex-1-enyl)phenol and reacting it with the product of Example 4a), 1.7g of the title compound were obtained as a colorless oil (37%) with a 96.5% purity (HPLC).

**¹H -NMR (DMSO-d₆, 200 MHz):** 7,35 (t., 1H,); 7,10 (t., 1H, NH); 6,98 and 694 (d., 2H,); 6,83 (s., 1H,); 3,98 (q., 2H, J=7 Hz, OCH₂); 3,16 (d, 2H, J=6,2 Hz, NCH₂); 2,69 (b.s., 2H); 2,51 (s, 2H, CH₂CO₂); 2,30-2,10 (m, 4H,); 1,98 (s, 6H, NCH₃); 1,70-1,60 (m, 4H); 1,60-1,20 (m, 10H); 1,15 (t, 3H, CH₃)

**¹³C-NMR (DMSO-d₆, 50 MHz):** 170,08 (1C, C=O); 156,86 (1C, C=O); 150,08 (1C, C=C-O); 144,56 (1C, COH-C=CH); 135,00 (1C, cyclohexene); 131,02 (1C, cyclohexene); 129,01 (1C, phenyl); 125,52 (1C, phenyl); 121,68 (1C, phenyl); 119,61 (1C, phenyl); 61,25 (1C, CH₂N); 59,60 (1C, CH₂O); 46,61 (1C, CH₂NH); 44,64 (2C, NCH₃); 39,50 (1C); 37,15 (1C); 32,54 (1C); 32,14 (1C); 27,10 (1C); 25,56 (1C); 22,79 (1C); 22,34 (1C); 21,03 (2C); 14,65 (1C, CH₃)

### Example 13. 3-(6-((dimethylamino)methyl)cyclohex-1-enyl)phenyl 2-(1-((ethoxy carbonylamino)methyl)cyclohexyl)acetate

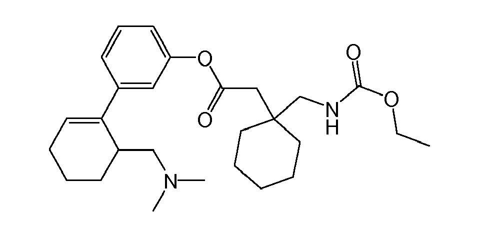

Following the procedure described in Example 1, but using as starting material 3-(6-((dimethylamino)methyl)cyclohex-1-enyl)phenol and reacting it with the product of Example 4a), 2.1g of the title compound were obtained as a colorless oil (46%) with a 93.4% purity (HPLC).

**¹H -NMR (DMSO-d₆, 200 MHz):** 7,35 (t., 1H,); 7,24 (d., 1H); 7,07 (b.s., 2H, 1Hphenyl+NH); 6,93 (d., 1H,); 6.02 (t, 1H, cyclohexene); 3,98 (q., 2H, J=7 Hz, OCH₂); 3,16 (d, 2H, J=6,2 Hz, NCH₂); 2,90 (b.s., 1H); 2,51 (s, 2H, CH₂CO₂); 2,30-2,10 (m, 3H,); 2,08 (s, 6H, NCH₃); 1,90-1,70 (m, 1H); 1,60-1,20 (m, 14H); 1,15 (t, 3H, CH₃)

**¹³C-NMR (DMSO-d₆, 50 MHz):** 170,08 (1C, C=O); 156,89 (1C, C=O); 150,51 (1C, C=C-O); 143,18 (1C, COH-C=CH); 138,66 (1C, cyclohexene); 129,22 (1C, phenyl); 127,54 (1C, cyclohexene); 122,95 (1C, phenyl); 119,96 (1C, phenyl); 119,03 (1C, phenyl); 61,34 (1C, CH₂N); 59,61 (1C, CH₂O); 46,63 (1C, CH₂NH); 45,33 (2C, NCH₃); 39,50 (1C); 37,19 (1C); 32,75 (1C); 32,51 (1C); 25,60 (1C); 25,53 (1C); 24,65 (1C); 24,46 (1C); 21,02 (2C); 17,12 (1C); 14,65 (1C, CH₃)

### Example 14. (1R2R, 1S2S)3-(1-chloro-2-((dimethylamino)methyl) cyclohexyl)phenyl 2-(1-((ethoxycarbonylamino)methyl)cyclohexyl)acetate

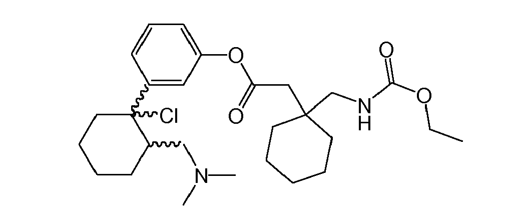

Following the procedure described in Example 1, but using as starting material (1R2R, 1S2S)3-(1-chloro-2-((dimethylamino)methyl)cyclohexyl)phenol and reacting it with the product of Example 4a), the title compound was obtained as a colorless oil.

**¹H -NMR (DMSO-d₆, 200 MHz):** 7,56 (d., 1H,); 7,42-7,30 (m., 2H,); 7,10-6,90 (m., 2H, 1Hphenyl+NH); 3,96 (q., 2H, J=7 Hz, OCH₂); 3,13 (d, 2H, J=6,2 Hz, NCH₂); 2,48 (s, 2H, CH₂CO₂); 2,40-2,20 (m, 1H,); 1,93 (s, 6H, NCH₃); 2,10-1,20 (m, 20H); 1,15 (t, 3H, CH₃)

**¹³C-NMR (DMSO-d₆, 50 MHz):** 169,99 (1C, C=O); 156,92 (1C, C=O); 150,27 (1C, C=C-O); 146,55 (1C, COH-C=CH); 129,10 (1C, phenyl); 123,44 (1C, phenyl); 120,80 (1C, phenyl); 119,84 (1C, phenyl); 81,44 (1C, C-C1); 61,30 (1C, CH₂N); 59,64 (1C, CH₂O); 46,62 (1C, CH₂NH); 45,64 (2C, NCH₃); 44,00 (1C); 43,84 (1C); 39,50 (1C); 37,16 (1C); 33,58 (2C); 25,56 (1C); 24,48 (2C); 22,47 (1C); 21,06 (2C); 14,68 (1C, CH₃)

### Example 15. 3-((2R,3S)-1-(dimethylamino)-2-methylpentan-3-yl)phenyl 2-(1-((ethoxycarbonylamino)methyl)cyclohexyl)acetate

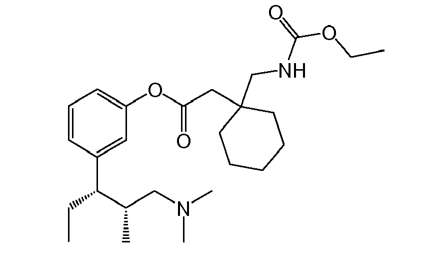

Following the procedure described in Example 1, but using as starting material 3-((2R,3S)-1-(dimethylamino)-2-methylpentan-3-yl)phenol and reacting it with the product of Example 4a), 2.15g of the title compound were obtained as a colorless oil with a 98.4% purity (HPLC).

**¹H -NMR (DMSO-d₆, 200 MHz):** 7,28 (t., 1H,); 7,20-6,95 (m., 2H, 1Hphenyl+NH); 6,90-6,80 (m., 2H); 3,97 (q., 2H, J=7 Hz, OCH₂); 3,14 (d, 2H, J=6,2 Hz, NCH₂); 2,49 (s, 2H, CH₂CO₂); 2,50-2,30 (m, 1H,); 2,03 (s, 6H, NCH₃); 2,00-1,60 (m, 5H); 1,60-1,20 (m, 10H); 1,15 (t, 3H, CH₃); 0,83 (d., 3H, CH₃); 0,64 (t, 3H, CH₃)

**¹³C-NMR (DMSO-d₆, 50 MHz):** 169,97 (1C, C=O); 156,86 (1C, C=O); 150,32 (1C, C=C-O); 145,77 (1C, COH-C=CH); 128,85 (1C, phenyl); 125,68 (1C, phenyl); 121,45 (1C, phenyl); 119,18 (1C, phenyl); 63,95 (1C, CH₂N); 59,58 (1C, CH₂O); 50,15 (1C); 46,63 (1C, CH₂NH); 45,45 (2C, NCH₃); 39,50 (1C); 37,14 (1C); 35,97 (1C); 32,58 (2C); 25,55 (1C); 23,08 (1C); 21,04 (2C); 18,53 (1C); 15,53 (1C); 14,65 (1C)

### Example 16. (1R2R, 1S2S)-(S)-3-(2-((dimethylamino)methyl)-1-hydroxycyclohexyl) phenyl 3-((ethoxycarbonylamino)methyl)-5-methylhexanoate

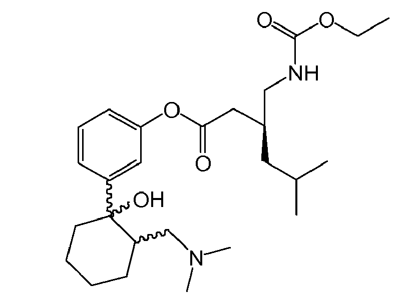

### 16a) Synthesis of (S)-3-((ethoxycarbonylamino)methyl)-5-methylhexanoic acid

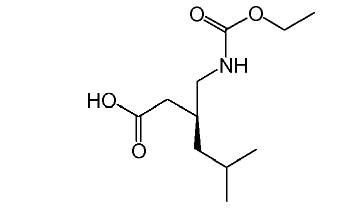

Following the procedure described in Example 4a, but using (S)-3-(aminomethyl)-5-methylhexanoic acid instead of 2-[1-(aminomethyl)cyclohexyl]acetic acid, the title compound was obtained in 95% yield.

**¹H -NMR (DMSO-d₆, 200 MHz):** 12,00 (b.s., 1H, CO₂H); 7,07 (b.s., 1H, NH); 3,95 (q., 2H, J=7 Hz, CH₂O); 3,10-2,70 (m, 2H, NNCH₂); 2,30-2,10 (m, 1H); 1,98 (s, 2H, CH₂CO₂); 1,70-1,50 (m, 1H); 1,20-0,90 (m, 5H); 0,88-0,79 (m, 6H, 2CH₃)

**¹³C-NMR (DMSO-d₆, 50 MHz):** 174,69 (1C, C=O); 157,25 (1C, C=O); 60,21 (1C, CO); 44,44 (1C, CH₂NH); 41,69 (1C, CH₂CO₂); 37,87 (1C); 33,89 (1C); 25,40 (1C); 23,54 (1C); 23,19 (1C); 15,38 (1C, CH₃)

### 16b) Synthesis of (1R2R, 1S2S)-(S)-3-(2-((dimethylamino)methyl)-1-hydroxy cyclohexyl)phenyl 3-((ethoxycarbonylamino)methyl)-5-methylhexanoate

Following the procedure described in Example 1, but using as starting material the product of Example 16a), 2.1g of the title compound were obtained as a colorless oil (45%) with a 99.2% purity (HPLC).

**¹H -NMR (DMSO-d₆, 200 MHz):** 7,31 (b.s., 2H,); 7,20-7,10 (m., 2H, 1Hphenyl+NH); 6,86 (b.s., 1H); 5-10-4,90 (b.s., 1H, OH); 3,98 (q., 2H, J=7 Hz, OCH₂); 3,20-2,80 (m, 2H, NCH₂); 2,60-2,30 (m, 2H); 2,20-2,00 (m, 2H); 1,88 (s, 6H, NCH₃); 2,00-1,30 (m, 11H); 1,20- 1,00 (m, 6H); 0.90-0.70 (m, 6H, 2CH₃)

**¹³C-NMR (DMSO-d₆, 50 MHz):** 171,16 (1C, C=O); 156,58 (1C, C=O); 151,72 (1C, C=C-O); 150,22 (1C, COH-C=CH); 128,53 (1C, phenyl); 122,22 (1C, phenyl); 119,03 (1C, phenyl); 118,41 (1C, phenyl); 74,43 (1C, C-OH); 60,33 (1C, CH₂N); 59,55 (1C, CH₂O); 45,86 (2C, NCH₃); 43,61 (1C, CH₂NH); 43,17 (1C); 40,99 (1C); 39,50 (1C); 36,95 (1C); 33,43 (1C); 26,38 (1C); 25,53 (1C); 24,70 (1C); 22,79 (1C); 22,48 (2C); 21,69 (1C); 14,66 (1C, CH₃)

### Example 17. (S)-3-((1R,2R)-2-((dimethylamino)methyl)-1-hydroxycydohexyl) phenyl 3-((ethoxycarbonylamino)methyl)-5-methylhexanoate

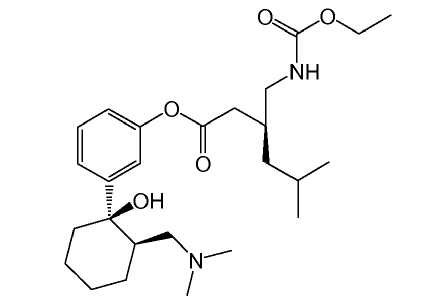

Following the procedure described in Example 1, but using as starting material the 1R2R isomer of 3-(2-((dimethylamino)methyl)-1-hydroxycyclohexyl)phenol and reacting it with the product of Example 16a), 2.2g of the title compound were obtained as a colorless oil (48%) with a 99.7% purity (HPLC) and 99.4% e.e.

**¹H -NMR (DMSO-d₆, 200 MHz):** 7,31 (b.s., 2H,); 7,20-7,10 (m., 2H, 1Hphenyl+NH); 6,86 (b.s., 1H); 5-10-4,90 (b.s., 1H, OH); 3,98 (q., 2H, J=7 Hz, OCH₂); 3,20-2,80 (m, 2H, NCH₂); 2,60-2,30 (m, 2H); 2,20-2,00 (m, 2H); 1,88 (s, 6H, NCH₃); 2,00-1,30 (m, 11H); 1,20-1,00 (m, 6H); 0.90-0.70 (m, 6H, 2CH₃)

**¹³C-NMR (DMSO-d₆, 50 MHz):** 171,16 (1C, C=O); 156,58 (1C, C=O); 151,72 (1C, C=C-O); 150,22 (1C, COH-C=CH); 128,53 (1C, phenyl); 122,22 (1C, phenyl); 119,03 (1C, phenyl); 118,41 (1C, phenyl); 74,43 (1C, C-OH); 60,33 (1C, CH₂N); 59,55 (1C, CH₂O); 45,86 (2C, NCH₃); 43,61 (1C, CH₂NH); 43,17 (1C); 40,99 (1C); 39,50 (1C); 36,95 (1C); 33,43 (1C); 26,38 (1C); 25,53 (1C); 24,70 (1C); 22,79 (1C); 22,48 (2C); 21,69 (1C); 14,66 (1C, CH₃)

### Example 18. (S)-3-((1S,2S)-2-((dimethylamino)methyl)-1-hydroxycyclohexyl)phenyl 3-((ethoxycarbonylamino)methyl)-5-methylhexanoate

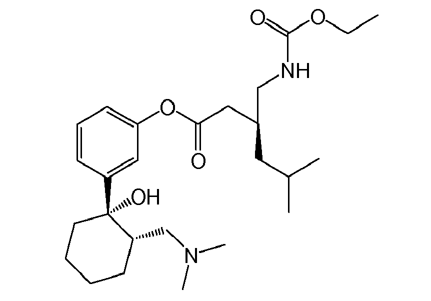

Following the procedure described in Example 1, but using as starting material the 1 S2S isomer of 3-(2-((dimethylamino)methyl)-1-hydroxycyclohexyl)phenol and reacting it with the product of Example 16a), 2.9g of the title compound were obtained as a colorless oil (63%).

**¹H -NMR (DMSO-d₆, 200 MHz):** 7,31 (b.s., 2H,); 7,20-7,10 (m., 2H, 1Hphenyl+NH); 6,86 (b.s., 1H); 5-10-4,90 (b.s., 1H, OH); 3,98 (q., 2H, J=7 Hz, OCH₂); 3,20-2,80 (m, 2H, NCH₂); 2,60-2,30 (m, 2H); 2,20-2,00 (m, 2H); 1,88 (s, 6H, NCH₃); 2,00-1,30 (m, 11H); 1,20-1,00 (m, 6H); 0.90-0.70 (m, 6H, 2CH₃)

**¹³C-NMR (DMSO-d₆, 50 MHz):** 171,16 (1C, C=O); 156,58 (1C, C=O); 151,72 (1C, C=C-O); 150,22 (1C, COH-C=CH); 128,53 (1C, phenyl); 122,22 (1C, phenyl); 119,03 (1C, phenyl); 118,41 (1C, phenyl); 74,43 (1C, C-OH); 60,33 (1C, CH₂N); 59,55 (1C, CH₂O); 45,86 (2C, NCH₃); 43,61 (1C, CH₂NH); 43,17 (1C); 40,99 (1C); 39,50 (1C); 36,95 (1C); 33,43 (1C); 26,38 (1C); 25,53 (1C); 24,70 (1C); 22,79 (1C); 22,48 (2C); 21,69 (1C); 14,66 (1C, CH₃)

### B) PHARMACOLOGY

### Description of the pharmacological processes

### Analgesic Activity Test

The pharmacological activity of the products of the present invention was tested *in vivo* in formalin test model, which is known to evaluate neuropathic and inflammatory pain in animals.

The activity of some of the products was also tested using an acute pain model by using the hot plate method.

### The Formalin Test: Characteristics and Usefulness of the Model

Sawynok, Jana; Liu, Xue Jun. Reviews in Analgesia, Volume 7, Number 2, 2003, pp. 145-163

The formalin test was introduced as a model of tonic pain in 1977, and has since been used extensively in rats and mice. In rats, formalin generates an initial phase of activity (5-10 min, phase 1), a quiescent interphase (5-10 min), and a second phase of activity (lasting 60-90 min, phase 2), and this is seen with spontaneous behaviors, firing of afferent neurons, and activity in dorsal horn neurons. Both active phases involve ongoing peripheral afferent neural activity; inflammation contributes to phase 2 activity and the interphase results from active inhibition. Responses are concentration dependent between 0.25% and 2.5%, plateau from 2.5% to 5%, and can decline at higher concentrations. Formalin also results in tissue edema, and this is longer lasting. Responses to formalin up to 2.5% are predominantly neurogenic, while at higher concentrations, responses involve a further prominent inflammatory component. Within the spinal cord, formalin increases c-Fos expression in neurons and causes activation of microglia, and these may contribute more prominently to longer term changes. Acute responses (to 90 min) may represent a model of ongoing acute pain involving inflammation and aspects of central sensitization, while longer term responses (days, weeks) may represent a model of changes involved in persistent pathological pain.

### Experimental Procedure

Male Swiss mice (weight range, 16-20 g) were used (n=6-8 per group).Mice were fasted for 3 hours before the administration of oral treatment with free access to drinking water before the experiment. All experiments were conducted in accordance with the "Guidelines on Ethical Standards for Investigation of Experimental Pain in Animals

Mice were housed in clear plastic chambers with a mirror placed at a 45° angle to allow an unobstructed view of the paws. Mice were injected into the dorsal surface of hind paw with 20 µl of dilute formalin (5%) using a 30-gauge needle. Treatments were administrated p.o. 10 min before formalin injection.

Animals were observed for nociceptive behavior immediately after formalin injection during 60 min. Nociceptive behavior was quantified measuring time spent licking the injected paw after injection.

For evaluation of antinociceptive effect of a potential drug for neuropathic pain, the quantization of the second phase of the test (15-60 min) is the most important. Controls animals were observed under identical conditions without receiving treatments before formalin injection.

For each dose of compound given the % inhibition of licking vs. control animals were calculated. ED 50 was calculated using the method of Litchfield and Wilcoxon. At the end of the experiments, mice were humanely euthanized with a dose of sodium pentobarbital (150 mg/kg i.p.).

### Hot plate method

The method that was used is described by Eddy N.B. and Leimbach D. (J. Pharm. Exp. Ther. 107: 385-393, 1953). The analgesic effect of the products was evaluated analyzing the behavior of the animals on a hot surface at 55°C±1°C.

Male Swiss mice weighing 20-25 g were used. The compounds being tested were administered orally 30 minutes before starting the test.

The process consisted of placing the animals on the plate and keeping them in a Plexiglas cylinder 25 cm high and 21 cm high. The time the animals took to jump off the hot surface was determined. The animals were selected before starting the test so that those that took longer than 10 seconds to jump off were not included in the group that would receive treatment.

30 minutes after administering the product being tested, the test was repeated and the maximum time it took the animals to jump off was again recorded. Those animals that did not jump off after 60 seconds were removed from the plate to avoid any injuries and were recorded as 100% protection.

The results are expressed as the % of jump time increase calculated as follows: %jump time increase = (treated jump time - base jump time) / basal jump time × 100

| PRODUCT | FORMALIN TEST (ED₅₀ p.o) | HOT PLATE (ED₅₀,p.o) |
|---|---|---|
| | Phase II | µmol/kg |
| | µmol/kg | |
| Tramadol hydrochloride | 138.7 | 66.0 |
| Tramadol Enantiomer R,R | 126.6 | n.d. |
| Tramadol Enantiomer S,S | 233.3 | n.d. |
| Product of example 1 | 39.9 | 35.7 |
| Product of example 2 | 17.2 | 8.0 |
| Product of example 3 | 65.9 | n.d. |
| Product of example 4 | 19.0 | 26.3 |
| Product of example 5 | 6.0 | n.d. |
| Product of example 6 | 28.0 | n.d. |
| Product of example 7 | 44.5 | n.d. |
| Product of example 8 | 57.9 | n.d. |
| Product of example 9 | 37.9 | n.d. |
| Product of example 10 | 39.0 | n.d. |
| Product of example 11 | 30.4 | n.d. |
| Product of example 12 | 62.7 | n.d. |
| Product of example 13 | 59.7 | n.d. |
| Product of example 15 | 30.4 | n.d. |

| PRODUCT | FORMALIN TEST (ED₅₀ p.o) | HOT PLATE (ED₅₀,p.o) |
|---|---|---|
| Product of example 16 | 75.2 | n.d. |
| Product of example 17 | 56.9 | n.d. |

## Claims

1. A compound of formula (I), or pharmaceutically acceptable, stereoisomers, salts
or solvates thereof 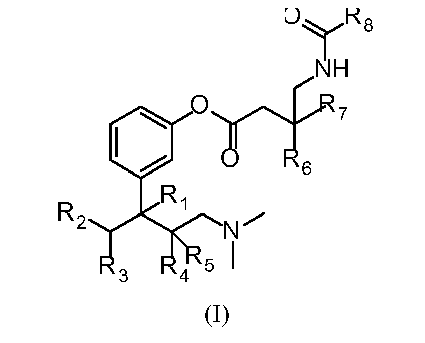 wherein
R₁ is selected from the group consisting of -H, -OH, -F, -Cl, -Br and -I;
R₂ is -H;
R₅ is -H;
or R₁ and R₂ together form a double bond and R₅ is -H
or R₁ and R₅ together form a double bond and R₂ is -H;
R₃ is a C₁-C₆ alkyl group;
R₄ is a C₁-C₆ alkyl group;
or R₃ and R₄ together form a C₂-C₄ alkylidene group;
R₆ is selected from the group consisting of -H and C₁-C₆ alkyl group;
R₇ is selected from the group consisting of -H and C₁-C₆ alkyl group;
or R₆ and R₇ together form a C₄-C₆ alkylidene group;
R₈ is selected from the group consisting of C₁-C₆ alkyl group, unsubstituted C₆-C₁₅ aryl, substituted C₆-C₁₅ aryl and O-R₉;
wherein R₉ is selected from the group consisting of C₁-C₆ alkyl group, C₆-C₁₀ aryl and C₆-C₁₀ aryl-C₁-C₂ alkyl.

2. A compound according to claim 1, wherein R₆ is hydrogen and R₇ is a C₁-C₆ alkyl group.

3. A compound according to claim 2, wherein R₇ is a branched C₁-C₆ alkyl group.

4. A compound according to claim 1, wherein R₆ and R₇ together form a C₄-C₆ alkylidene group.

5. A compound according to claim 4, wherein R₆ and R₇ form a cyclohexyl group together with the carbon atom to which they are attached.

6. A compound according to any of claims 1-5, wherein R₈ is a methyl group, a C₂-C₄ alkoxy group, a phenoxy group or a benzoxy group.

7. A compound according to claim 6 wherein R₈ is an ethoxy group.

8. A compound according to any of claims 1-7, wherein R₁ is hydroxyl.

9. A compound according to any of claims 1-7 of formula (IA), or pharmaceutically acceptable, stereoisomers, salts or solvates thereof 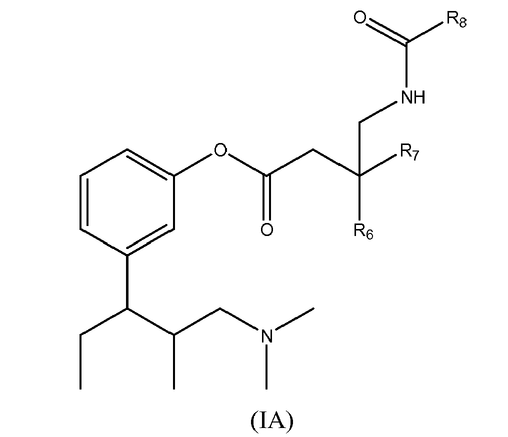 wherein R₆, R₇ and R₈ are as defined in the previous claims.

10. A compound according to claims 1-8 of formula (IB), or pharmaceutically acceptable, stereoisomers, salts or solvates thereof 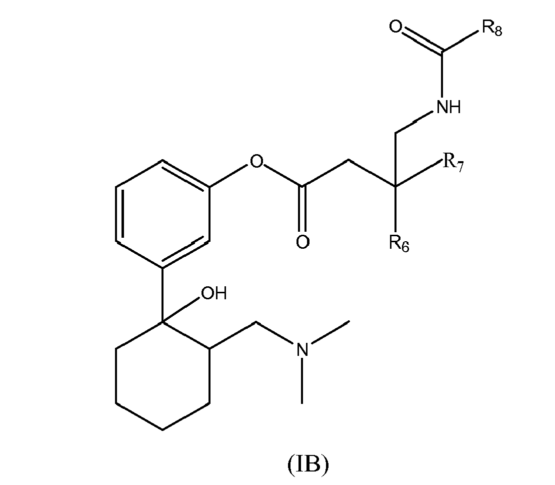 wherein R₆, R₇ and R₈ are as defined in the previous claims.

11. A compound according to claims 1-7 of formula (IC), or pharmaceutically acceptable, stereoisomers, salts or solvates thereof 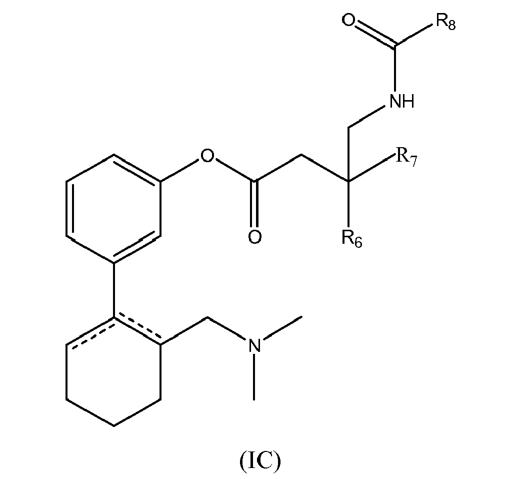 wherein R₆, R₇ and R₈ are as defined in the previous claims, and one of the dotted lines may represent a double bond.

12. A compound as defined in any of claims 1-11 for use as a medicament.

13. Pharmaceutical composition comprising a compound as defined in any of claims 1-11 and a pharmaceutically acceptable carrier.

14. Compound as defined in any of claims 1-11 for use in the treatment of pain.

15. Compound as defined in claim 14, wherein pain is chronic pain.

16. Compound as defined in claim 14 or 15, wherein pain is neurophatic pain.
